# EUROPEAN PATENT APPLICATION

(11) **EP 2 386 249 A1**
(43) Date of publication of application: **16.11.2011**
(21) Application number: 11163083.6
(22) Date of filing: 19.04.2011
(51) Int. Cl.: A61B 8/00

(54) **Ultrasonic diagnostic apparatus**

(30) Priority: 14.05.2010 KR 20100045276
(71) Applicant: Samsung Medison Co., Ltd., Nam-myun Hongchun-gun Kangwon do 250-875 (KR)
(72) Inventor: Hyoun, Dong Gyu, Gyeonggi-do (KR); Kim, Jong Sik, Seoul (KR)
(74) Representative: Schmid, Wolfgang

(57) **Abstract**

The present disclosure provides an ultrasonic diagnostic apparatus. The ultrasonic diagnostic apparatus includes a fastening part (20) having a band shape and wound around a diagnosis object (10); and an examination part (30) connected to the fastening part (20) and allowing a probe (12) to move in a different direction from a winding direction of the fastening part (20) around the diagnosis object. During ultrasonic diagnosis, with the fastening part (20) wound around a diagnosis object (10), the probe (12) moves in a different direction from a winding direction of the fastening part (20) around the diagnosis object (10) and provides uniform ultrasound images of the object, thereby improving reliability of the ultrasonic diagnosis.

## Description

### BACKGROUND

### 1. Technical Field

The present disclosure relates to an ultrasonic diagnostic apparatus and, more particularly, to an ultrasonic diagnostic apparatus which allows a probe to move along the length of a diagnosis object having a rounded surface or protruded portion so as to facilitate ultrasound-based diagnosis on the rounded surface or protruded portion of the object.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal).

The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a probe which transmits an ultrasound signal to a diagnosis object and receives the ultrasound signal reflected therefrom to obtain ultrasound images of the diagnosis object. A controller displays the signal sent from the probe on a display screen, so that a user can diagnose the object while alternately viewing the screen and the object.

It should be noted that the above description is provided for understanding of the background art and is not a description of a well-known conventional technique to which the present disclosure pertains.

Since some diagnosis objects such as the arm, the torso or the leg have rounded circumferences, a user of the ultrasonic diagnostic apparatus grips and moves the probe on a diagnosis object to scan the object. Such manipulation of the probe can provide nonuniform ultrasound images depending on operator skill, thereby lowering reliability of ultrasonic diagnosis. Moreover, since a user must move the probe along a rounded surface or protruded portion of the diagnosis object, the user may experience wrist strain. Therefore, there is a need for an improved ultrasonic diagnostic apparatus.

### BRIEF SUMMARY

The present disclosure is directed to solving such problems of the related art, and an aspect of the present disclosure is to provide an ultrasonic diagnostic apparatus which guarantees reliability in diagnosis of an object regardless of operator skill.

Another aspect of the present disclosure is to provide an ultrasonic diagnostic apparatus which may reduce user fatigue.

In accordance with one aspect, an ultrasonic diagnostic apparatus includes: a fastening part having a band shape and wound around a diagnosis object; and an examination part connected to the fastening part and allowing a probe to move in a different direction than a winding direction of the fastening part around the diagnosis object.

The fastening part may include a first band member connected to one side of the examination part and a second band member connected to the other side of the examination part.

The fastening part may further include a first fastener connected to the first band member and a second fastener connected to the second band member to be fastened to the first fastener.

The first and second fasteners may be fabric hook-and-loop fasteners.

The examination part may include an ultrasound permeable film connected to the fastening part and a mover moving the probe.

The apparatus may further include a gel pad disposed on one side of the ultrasound permeable film facing the diagnosis object and having a gel inside the gel pad.

The examination part may include a resilient gel pad connected to the fastening part and disposed in a moving direction of the probe, and a mover moving the probe inside the gel pad.

The mover may include a drive member supplying rotational power, a conveyor belt moved by rotation of the drive member, and a movable mounting part moved in conjunction with the conveyor belt and having the probe securely mounted on one side of the movable mounting part.

The mover may further include a drive gear powered by the drive member and supporting one side of the conveyor belt, and a driven gear supporting the other side of the conveyor belt.

The conveyor belt may be disposed on either side of the movable mounting part.

The mover may include a gear rack disposed on the ultrasound permeable film in a longitudinal direction in which the probe is moved, and a movable examination part moving along the gear rack and having the probe secured to a lateral side of the movable examination part.

The gear rack may be disposed on either side of the movable examination part.

The movable examination part may further include a pinion gear engaging with the gear rack, a transport motor driving the pinion gear, and a movable plate on which the transport motor, pinion gear and probe are mounted.

The movable examination part may include an anti-separation ring bent at opposite sides of the movable plate to catch lateral sides of the ultrasound permeable film.

### BRIEF DESCRIPTION OF THE DRAWINGS

The above and other features and advantages of the present disclosure will become apparent from the following description of exemplary embodiments given in conjunction with the accompanying drawings, in which:
Fig. 1 is a perspective view of an ultrasonic diagnostic apparatus according to one exemplary embodiment of the present disclosure in use;
Fig. 2 is a perspective view of the ultrasonic diagnostic apparatus according to the exemplary embodiment of the present disclosure;
Fig. 3 is an exploded perspective view of the ultrasonic diagnostic apparatus according to the exemplary embodiment of the present disclosure;
Fig. 4 is a perspective view of the ultrasonic diagnostic apparatus according to the exemplary embodiment of the present disclosure, showing a movable mounting part moving on a conveyor belt in Fig. 3;
Fig. 5 is a perspective view of the ultrasonic diagnostic apparatus according to the exemplary embodiment of the present disclosure, showing the conveyor belt mounted at an angle on an ultrasound permeable film;
Fig. 6 is an exploded perspective view of a mover of the ultrasonic diagnostic apparatus according to the exemplary embodiment of the present disclosure;
Fig. 7 is a block diagram of the ultrasonic diagnostic apparatus according to the first exemplary embodiment of the present disclosure;
Fig. 8 is an exploded perspective view of an ultrasonic diagnostic apparatus according to another exemplary embodiment of the present disclosure;
Fig. 9 is a perspective view of a mover in the ultrasonic diagnostic apparatus according to the other exemplary embodiment of the present disclosure;
Fig. 10 is an exploded perspective view of an ultrasonic diagnostic apparatus according to a further exemplary embodiment of the present disclosure; and
Fig. 11 is a plan view of the ultrasonic diagnostic apparatus of Fig. 10.

### DETAILED DESCRIPTION

Exemplary embodiments of the present disclosure will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the present disclosure into account and can be changed according to the custom or intention of users or operators. Therefore, definition of the terms should be made according to overall disclosures set forth herein.

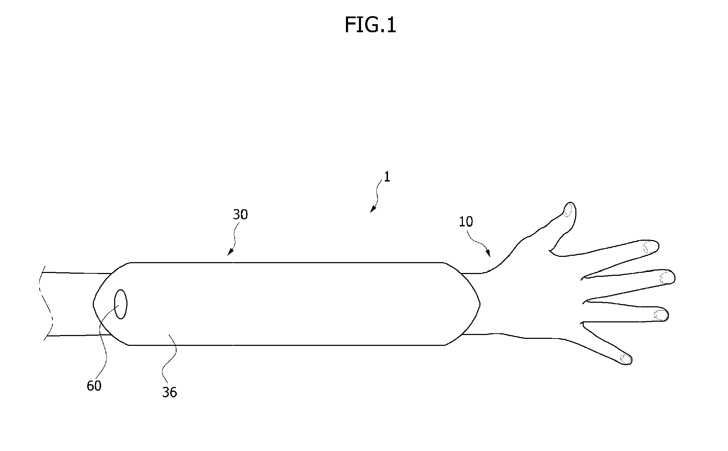

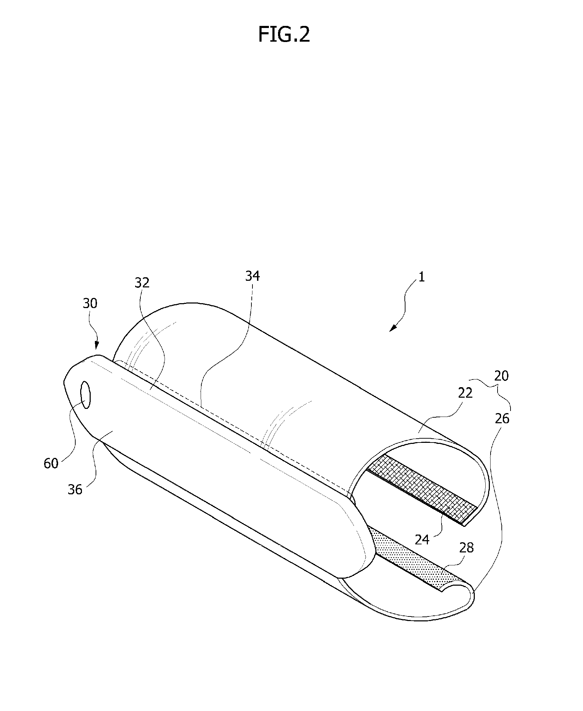

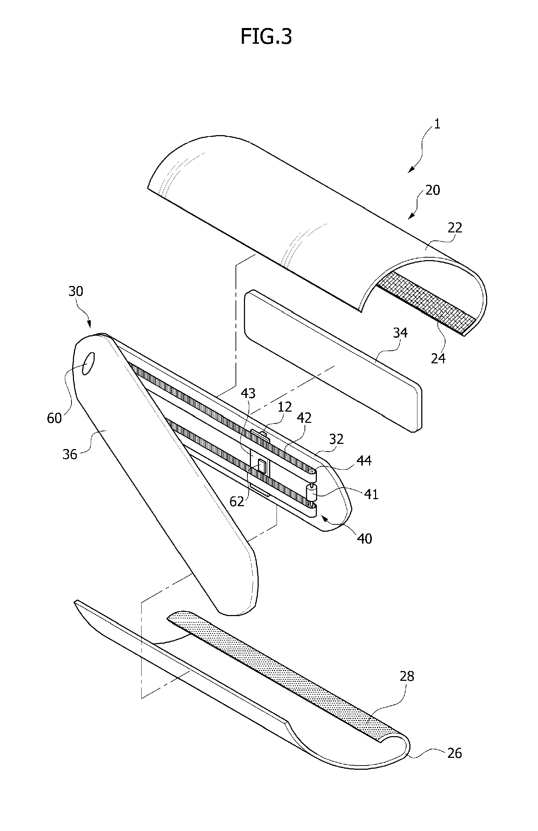

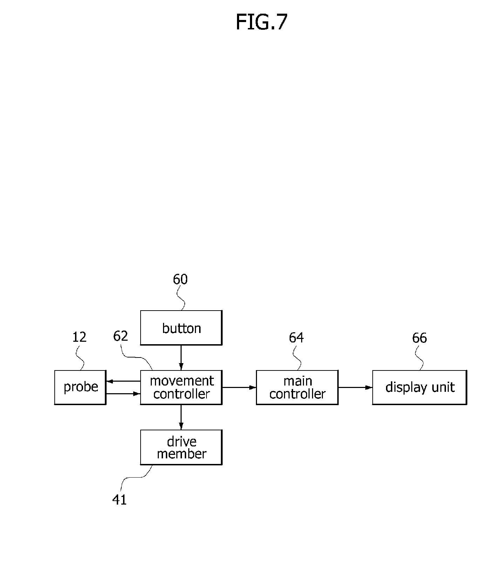

Figs. 1 to 7 show an ultrasonic diagnostic apparatus according to one exemplary embodiment of the present disclosure.

Referring to Figs. 1 to 7, the ultrasonic diagnostic apparatus 1 according to the exemplary embodiment includes a fastening part 20 which has a band shape and is wound around a diagnosis object 10, and an examination part 30 which is connected to the fastening part 20 and allows a probe 12 to move in a different direction from a winding direction of the fastening part 20 around the diagnosis object 10.

Herein, an arm of a person will be illustrated as the diagnosis object 10. Since the apparatus 1 allows the probe 12 to move on the diagnosis object 10 with the fastening part 20 wound around the diagnosis object 10, the apparatus 1 may provide various ultrasound images of the diagnosis object 10.

Any device may be used as the probe 12 for the ultrasonic diagnostic apparatus 1 so long as the device can move in a different direction from the winding direction of the fastening part 20 around the diagnosis object 10 while transmitting or receiving ultrasound signals to obtain the ultrasound images of the diagnosis object 10.

The fastening part 20 for fastening the ultrasonic diagnostic apparatus 1 to the object 10 includes a first band member 22 connected to one side of the examination part 30 and a second band member 26 connected to the other side of the examination part 30.

Since the fastening part 20 is securely wound around the object 10, the fastening part 20 restricts movement of the examination part 30, and any stretchable member such as an stretchable string or band may be used as the fastening part 20 so long as the stretchable member can provide such functions of the fastening part 20.

The fastening part 20 further includes a first fastener 24 connected to the first band member 22, and a second fastener 28 connected to the second band member 26 to be fastened to the first fastener 24. Any members may be used as the first and second fasteners 24, 28 so long as the members can be fastened to each other in this manner.

In this embodiment, fabric hook-and-loop fasteners such as Velcro fasteners are used as the first and second fasteners 24, 28, thereby allowing easy adjustment in length and easy attachment/detachment of the fastening part 20 to the diagnosis object 10.

When the fastening part 20 is wound around the diagnosis object 10, the examination part 30 is disposed adjacent the object 10 and allows the probe 12 to move in a different direction from the winding direction of the fastening part 20 around the object 10 to obtain ultrasound images of the object 10. Any measuring device may be used as the examination part 30 so long as the device can be operated in this manner to provide the ultrasound images of the diagnosis object.

In this embodiment, the examination part 30 includes an ultrasound permeable film 32 connected to the fastening part 20 and formed of a material allowing ultrasound waves to pass therethrough, and a mover 40 which moves the probe 12.

The ultrasound permeable film 32 may be made of any stretchable material that has high permeability to ultrasound waves and can be bent on the object 10.

A gel pad 34 may be disposed adjacent the ultrasound permeable film 32. Here, it should be understood that the ultrasound permeable film 32 may be used without the gel pad 34.

The gel pad 34 is provided to one side of the ultrasound permeable film 32 facing the diagnosis object 10 and includes an ultrasound permeable gel therein.

When the probe 12 moves along the diagnosis object 10, the gel pad 34 is in close contact with the diagnosis object 10 along a circumference of the object 10, thereby preventing measurement error caused by formation of a gap between the probe 12 and the object 10.

Further, since the gel pad 34 of a predetermined thickness is disposed between the diagnosis object 10 and the probe 12, the gel pad 34 may prevent any possible diffuse reflection resulting from closeness between the probe 12 and the diagnosis object 10, thereby improving reliability of the ultrasound images.

The mover 40 provided to the ultrasound permeable film 32 moves the probe 12 in the different direction from the winding direction of the fastening part 20 around the diagnosis object 10 to obtain ultrasound images of the diagnosis object 10. Any device may be used as the examination part 30 so long as the device can move the probe 12 in this manner.

For example, the fastening part 20 is wound around an arm of a person as the diagnosis object 10 and the probe 12 moves on the arm in the longitudinal direction of the arm to obtain ultrasound images of the arm.

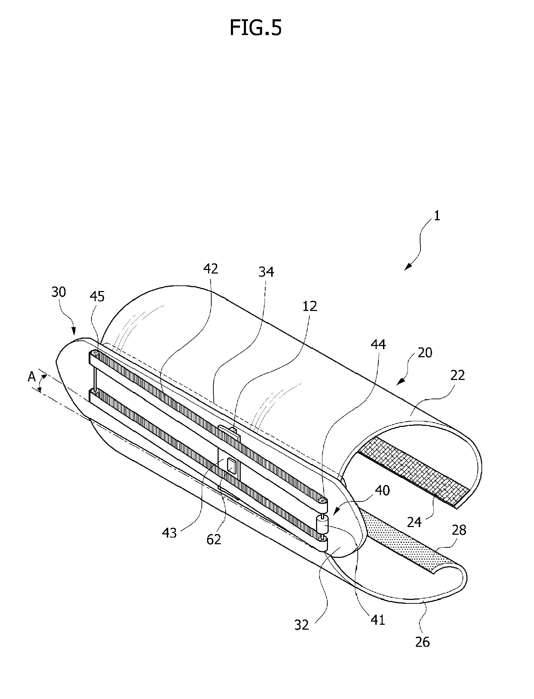

The winding direction of the fastening part 20 may be perpendicular to the moving direction of the probe 12. Alternatively, as shown in Fig. 5, when conveyor belts 42 are installed at a preset angle A on the ultrasound permeable film 32, the probe 12 may move diagonally along the ultrasound permeable film 32. As such, the moving direction of the probe 12 may be changed in various ways.

According to this embodiment, the mover 40 includes a drive member 41 supplying rotational power, the conveyor belts 42 moved by rotation of the drive member 41, a movable mounting part 43 moved in conjunction with the conveyor belts 42 and having the probe 12 secured to one side thereof, and a drive gear 44 powered by the drive member 41 and supporting one side of each of the conveyor belts 42, and a driven gear 45 supporting the other side of each of the conveyor belts 45.

The drive member 41 includes a motor for supplying the rotational power and is secured to the ultrasound permeable film 32.

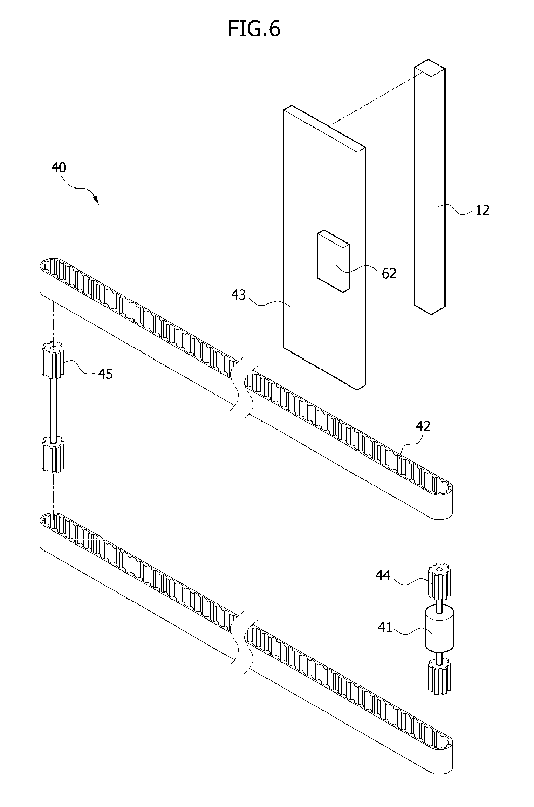

The drive gear 44 is coupled to either side of a shaft extending in both directions through the drive member 41 (see Fig. 6).

The drive gear 44 supports the one side (right side in Fig. 6) of each conveyor belt 42 and the driven gear 45 supports the other side of each conveyor belt 42.

The driven gear 45 is also coupled to either side of a connection bar, which connects both driven gears 45 such that the driven gears 45 are rotated at the same time.

The conveyor belt 42 has a threaded inner surface, which engages with the drive gear 44 and driven gear 45 to rotate therewith.

The movable mounting part 43 having the probe 12 mounted thereon is secured to the conveyor belts 42 and moved in conjunction with the conveyor belts 42.

Since the conveyor belts 42 are disposed at opposite sides of the movable mounting part 43, the movable mounting part 43 may be more stably moved in a longitudinal direction of the conveyor belts 42 (see Fig. 6).

The movable mounting part 43 is provided with a movement controller 62 which controls operation of the drive member 41 and probe 12.

The movement controller 62 is controlled by a button 60, which may be disposed on a cover 36 covering the ultrasound permeable film 32 or on other parts of the diagnostic apparatus.

The movement controller 62 is connected to a main controller 64 via wired or wireless communication to send an ultrasound signal of the probe 12 thereto, and the main controller 64 converts the ultrasound signal into an image signal and sends the image signal to a display unit 66.

The mover 40 is operated by manipulation of the button 60 on the cover 36 and the drive member 41 receives power via wired or wireless communication. Alternatively, the drive member 41 may receive power from a battery inside the examination part 30.

Next, operation of the ultrasonic diagnostic apparatus according to the first exemplary embodiment will be described with reference to the accompanying drawings.

With the fastening part 20 wound around a diagnosis object 10, the first fastener 24 is fastened to the second fastener 28 to secure the fastening part 20 to the object 10. As the fastening part 20 is secured to the object 10, the examination part 30 including the probe 12 is also disposed adjacent the object 10.

When a user presses the button 60, power is supplied to the drive member 41 to generate rotational power, which is transmitted to and rotates the drive gears 44 located at opposite sides of the drive member 41.

Rotation of the drive gears 44 leads to rotation of the conveyor belts 42 between the drive gears 44 and the driven gears 45.

Then, the movable mounting part 43 secured to the conveyor belts 42 is also moved together with the conveyor belts 42 and the probe 12 mounted on the movable mounting part 43 is moved while scanning the diagnosis object 10 to generate ultrasound images thereof.

Since the gel pad 34 attached to the rear of the ultrasound permeable film 32 is disposed to surround the diagnosis object 10 having a curved shape, it is possible to reduce a measurement error caused when pockets of air are formed between the probe 12 and the diagnosis object 10 during ultrasonic diagnosis.

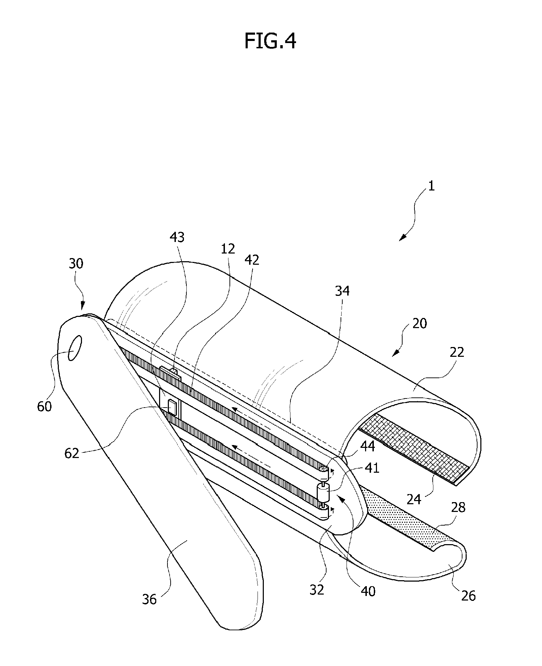

The movement controller 62 calculates a movement location of the probe 12 and adjusts rotation of the drive member 41 in a forward or rearward direction to move the probe 12 in a side to side direction (see Fig. 4).

An ultrasound signal from the probe 12 is transmitted to the main controller 64 via the movement controller 62. Then, the main controller 62 converts the ultrasound signal into an image signal and sends the image signal to a display unit 66, thereby providing an ultrasound image of the diagnosis object 10.

Next, an ultrasonic diagnostic apparatus 2 according to another exemplary embodiment will be described with reference to the accompanying drawings.

For convenience of description, the same elements as those of the above embodiment will be de denoted by the same reference numerals and a detailed description thereof will be omitted herein.

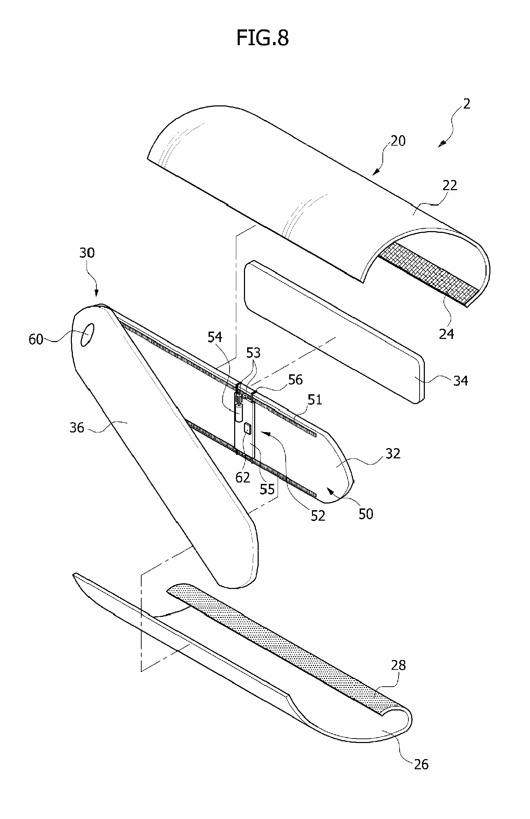

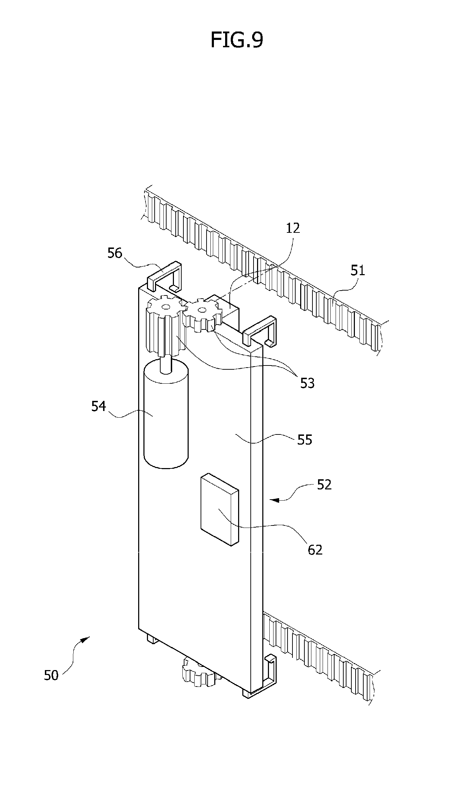

Figs. 8 and 9 schematically show the ultrasonic diagnostic apparatus 2 according to the other exemplary embodiment.

Referring to Figs. 8 and 9, in this embodiment, a mover 50 includes gear racks 51 disposed on a fastening part 20 in a longitudinal direction in which a probe 12 is moved, and a movable examination part 52 moving along the gear racks 51 and having the probe 12 secured to a lateral side of the movable examination part 52.

The gear racks 51 are secured to opposite sides of an ultrasound permeable film 32, respectively, and both of the gear racks 51 and ultrasound permeable film 32 are made of a flexible material.

Since the gear racks 51 are disposed at the opposite sides of the movable examination part 52 including the probe 12 (see Fig. 8), the movable examination part 52 may be more stably moved in a longitudinal direction of the gear racks 51.

In this embodiment, the movable examination part 52 includes pinion gears 53, which engage with and rotate on the gear racks 51, a transport motor 54 driving the pinion gears 53, a movable plate 55 on which the transport motor 54, pinion gears 53 and probe 12 are mounted, and anti-separation rings 56 each bent at opposite sides of the movable plate 55 to catch lateral sides of the fastening part 20.

Each of the anti-separation rings 56 separated from each other extends across the movable plate 55 and is bent at the opposite sides of the movable plate 55. Since both sides of each of the anti-separation rings 56 extend to and catch the rear side of the ultrasound permeable film 32, the anti-separation rings 56 prevent the movable plate 55 from being separated from the movable examination part 52.

The movable plate 55 is provided with a movement controller 62, which controls operation of the transport motor 54 which rotates the pinion gears 53 engaging with the gear racks 51.

One of the pinion gears 53 may be disposed under the movable plate 55 to engage with and rotate on the gear rack 51.

Next, operation of the ultrasonic diagnostic apparatus according to this embodiment will be described with reference to the accompanying drawings.

When a user presses a button 60, power is supplied to the transport motor 54 to generate rotational power, which is transmitted to and rotates the pinion gears 53 connected to the transport motor 54.

Since the pinion gears 53 engages with and rotate on the gear racks 51, the movable plate 55 including the probe 12 is horizontally moved along the gear racks 51 (see Fig. 8).

The probe 12 attached to the movable plate 55 is also horizontally moved while scanning a diagnosis object 10 to generate ultrasound images thereof.

The movement controller 62 calculates a movement location of the probe 12 and adjusts rotation of the transport motor 54 in a forward or rearward direction to move the probe 12 in a side-to-side direction (see Fig. 8).

Next, an ultrasonic diagnostic apparatus 3 according to a further exemplary embodiment will be described with reference to the accompanying drawings.

For convenience of description, the same elements as those of the above exemplary embodiment will be de denoted by the same reference numerals and a detailed description thereof will be omitted herein.

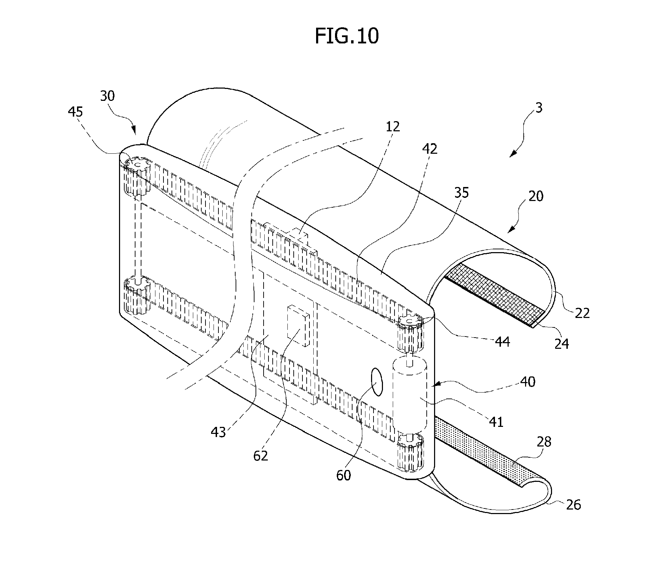

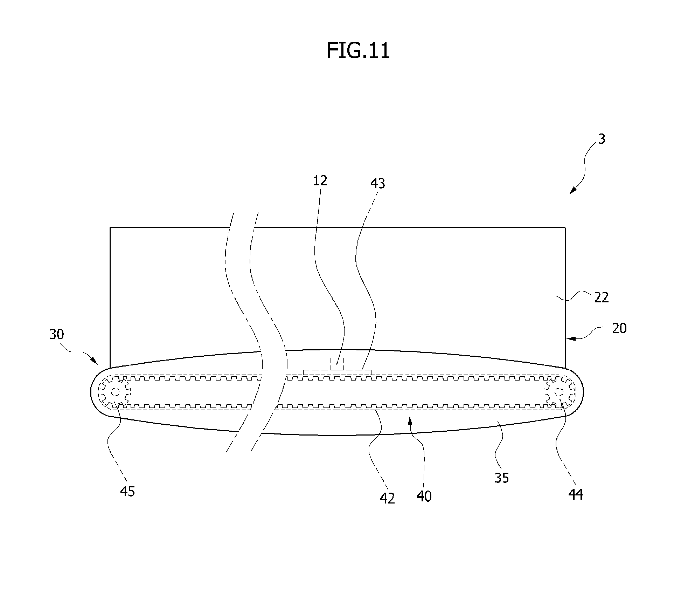

Figs. 10 and 11 schematically show the ultrasonic diagnostic apparatus 3 according to the further embodiment.

Referring to Figs. 10 and 11, in this embodiment, an examination part 30 includes a resilient gel pad 35 connected to the fastening part 20 and disposed in a moving direction of a probe 12, and a mover 40 moving the probe 12 inside the gel pad 35.

In this embodiment, the probe 12 and the mover 40 for automatically moving the probe 12 are disposed inside the gel pad 35. The gel pad 35 is in close contact with a diagnosis object 10 to prevent a gap from being formed between the probe 12 and the diagnosis object 10. Any member may be used as the gel pad 35 so long as the member can provide such a function of the gel pad 12 according to this embodiment.

The fastening part 20 includes a first band member 22 and a second band member 26 disposed at opposite sides of the gel pad 35, respectively.

A drive member 41, drive gears 44 and driven gears 45 are disposed outside or inside the gel pad 35 to rotate conveyor belts 42.

The ultrasonic diagnostic apparatus 3 according to this embodiment omits the ultrasound permeable film 32 and the cover 36 of the embodiment described above, thereby reducing manufacturing costs.

Next, operation of the ultrasonic diagnostic apparatus 3 according to this embodiment will be described with reference to the accompanying drawings.

When a user presses a button 60 inside the gel pad 35, power is supplied to the drive member 41 to generate rotational power, which is transmitted to and rotates the drive gears 44 located at opposite sides of the drive member 41 (see Fig. 10).

Rotation of the drive gears 44 leads to rotation of the conveyor belts 42 between the drive gears 44 and the driven gears 45 inside the gel pad 35.

Then, a movable mounting part 43 secured to the conveyor belts 42 is moved together with the conveyor belts 42 inside the gel pad 35.

The probe 12 attached to the movable mounting part 43 is also horizontally moved while scanning the diagnosis object 10 to generate ultrasound images thereof. Here, since only an ultrasound permeable gel is disposed between the probe 12 and the object 10, it is possible to reduce measurement errors caused by pockets of air therebetween.

The movement controller 62 calculates a movement location of the probe 12 and adjusts rotation of the drive member 41 in a forward or rearward direction so as to allow the probe 12 to move only inside the gel pad 35.

Accordingly, in each of the ultrasonic diagnostic apparatuses 1, 2, 3 according to the embodiments described above, the probe 12 provides uniform ultrasound images of the diagnosis object 30 while moving along the examination part 30 securely wound around the diagnosis object 10, so that the ultrasonic diagnostic apparatus may guarantee improved reliability of ultrasonic diagnosis.

In addition, since the probe 12 is automatically moved on the fastening part, the ultrasonic diagnostic apparatus may guarantee improved operability by reducing user fatigue.

As such, according to the embodiments, the ultrasonic diagnostic apparatus includes the fastening part wound around a diagnosis object and allows the probe to move in a different direction from the winding direction of the fastening part around the object to provide uniform ultrasound images of the object, thereby improving reliability of ultrasonic diagnosis.

In addition, the ultrasonic diagnostic apparatus may reduce user fatigue by allowing automatic movement of the probe, thereby enhancing operability during ultrasonic diagnosis.

In addition, the probe is secured around the diagnosis object via fabric hook-and-loop fasteners, strings or bands during scanning, instead of a user directly grabbing the probe to secure the probe to the object, thereby reducing user fatigue.

Further, the ultrasound permeable film may be flexibly bent and the gear rack may also be bent in the same direction as that of the ultrasound permeable film, so that the probe provided to the movable examination part can scan the diagnosis object along the circumference of the object without resistance.

Further, the ultrasonic diagnostic apparatus allows the probe to move on a diagnosis object in the longitudinal direction of the ultrasound permeable film, so that the probe can contact a wide region despite curvatures of the diagnosis object, thereby guaranteeing a wide diagnosis area.

Furthermore, the ultrasonic diagnostic apparatus allows scanning of the diagnosis object without direct manual user manipulation and thus provides uniform ultrasound images of patients, thereby enhancing reliability of ultrasonic diagnosis.

Although the present disclosure has been described with reference to the embodiments shown in the drawings, it should be understood by those skilled in the art that these embodiments are given by way of illustration only, and that various modifications, variations, and alternations can be made without departing from the spirit and scope of the present disclosure. Further, although an arm of a person is illustrated as a diagnosis object 10, the ultrasonic diagnostic apparatus according to the present disclosure is obviously applied to other parts of a person for ultrasonic diagnosis. The scope of the present disclosure should be limited only by the accompanying claims and equivalents thereof.

## Claims

1. An ultrasonic diagnostic apparatus (1; 2; 3) **characterized by** comprising:
a fastening part (20) having a band shape and wound around a diagnosis object (10); and
an examination part (30) connected to the fastening part (20) and allowing a probe (12) to move in a different direction from a winding direction of the fastening part (20) around the diagnosis object (10).

2. The apparatus (1; 2; 3) according to claim 1, **characterized in that** the fastening part (20) comprises a first band member (22) connected to one side of the examination part (30) and a second band member (26) connected to the other side of the examination part (30).

3. The apparatus (1; 2; 3) according to claim 2, **characterized in that** the fastening part (20) further comprises a first fastener (24) connected to the first band member (22), and a second fastener (28) connected to the second band member (26) to be fastened to the first fastener (24).

4. The apparatus (1; 2; 3) according to claim 3, **characterized in that** the first and second fasteners (24, 28) are fabric hook-and-loop fasteners.

5. The apparatus (1; 2; 3) according to claim 1, **characterized in that** the examination part (30) comprises an ultrasound permeable film (32) connected to the fastening part (20) and a mover (40; 50) moving the probe (12).

6. The apparatus (1; 2; 3) according to claim 5, **characterized by** further comprising:
a gel pad (34) disposed on one side of the ultrasound permeable film (32) facing the diagnosis object (10) and having a gel inside the gel pad (34).

7. The apparatus (1) according to claim 5, **characterized in that** the mover (40) comprises:
a drive member (41) supplying rotational power;
a conveyor belt (42) moved by rotation of the drive member (41); and
a movable mounting part (43) moved in conjunction with the conveyor belt (42) and having the probe (12) securely mounted to one side of the movable mounting part (43).

8. The apparatus (1) according to claim 7, **characterized in that** the mover (40) further comprises:
a drive gear (44) powered by the drive member (41) and supporting one side of the conveyor belt (42); and
a driven gear (45) supporting the other side of the conveyor belt (42).

9. The apparatus (1) according to claim 7, **characterized in that** the conveyor belt (42) is disposed on either side of the movable mounting part (43).

10. The apparatus (2) according to claim 5, **characterized in that** the mover (50) comprises:
a gear rack (51) disposed on the ultrasound permeable film (32) in a longitudinal direction in which the probe (12) is moved; and
a movable examination part (52) moving along the gear rack (51) and having the probe (12) secured to a lateral side of the movable examination part (52).

11. The apparatus (2) according to claim 10, **characterized in that** the gear rack (51) is disposed on either side of the movable examination part (52).

12. The apparatus (2) according to claim 10, **characterized in that** the movable examination part (52) comprises:
a pinion gear (53) engaging with the gear rack (51) to rotate thereon;
a transport motor (54) driving the pinion gear (53); and
a movable plate (55) on which the transport motor (54), pinion gear (53) and probe (12) are mounted.

13. The apparatus (2) according to claim 12, **characterized in that** the movable examination part (52) further comprises an anti-separation ring (56) bent at opposite sides of the movable plate (55) to catch lateral sides of the ultrasound permeable film (32).

14. The apparatus (3) according to claim 1, **characterized in that** the examination part (30) comprises:
a resilient gel pad (35) connected to the fastening part (20) and disposed in a moving direction of the probe (12); and
a mover (40) moving the probe (12) inside the gel pad (35).
